# EUROPEAN PATENT APPLICATION

(11) **EP 4 375 805 A1**
(43) Date of publication of application: **29.05.2024**
(21) Application number: 22209745.3
(22) Date of filing: 25.11.2022
(51) Int. Cl.: G06F 3/01, A61B 5/16

(54) **AUDIO OUTPUT**

(71) Applicant: Nokia Technologies Oy, 02610 Espoo (FI)
(72) Inventor: QENDRO, Lorena, Cambridge CB1 8DZ (GB); JANG, Si Young, Seoul 06182 (KR); FERLINI, Andrea, Cambridge CB4 3QF (GB)
(74) Representative: Whiting, Gary

(57) **Abstract**

An apparatus, method and computer program is described comprising: capturing imaging data relating to a user environment; providing captured imaging data to a processor for generating content based, at least in part, on the captured imaging data, wherein the generated content comprises audio content; receiving said generated content; and providing an audio output to the user based on said generated content.

## Description

### Field

The present specification relates to providing audio output to a user.

### Background

The provision of audio content (such as autonomous sensory meridian response (ASMR) content) is known. There remains a need for further developments in the field, for example in relation to navigating the wide range of available content.

### Summary

In a first aspect, this specification describes an apparatus comprising: means for capturing imaging data relating to a user environment; means for obtaining (e.g. receiving or retrieving) user information; means for providing at least the captured imaging data to a processor (e.g. a processor implementing a ML model trained in accordance with the principles described herein) for generating content based, at least in part, on the captured imaging data, wherein the generated content comprises audio content; means for receiving said generated content; and means for providing an audio output to the user based on said generated content, wherein the audio output is dependent, at least in part, on the captured imaging data and the user information. The apparatus may be, or may form part of, an eyewear device (such as smart glasses or the like). The audio content may be modified (e.g. personalised) in some way. In some example embodiments, the audio content is intended to induce an autonomous sensory meridian response (ASMR).

Some example embodiments further comprise means for obtaining information relating to a mood state of the user, wherein the output provided to the user is based, at least in part, on said mood state information. Thus, the output (e.g. audio output) provided to the user may be personalised based (at least in part) on said mood state information. Such modification maybe performed at the apparatus (e.g. at an eyewear device), at a processor of a connected user device (such as a mobile phone) or elsewhere (e.g. at a remote processor, such as "cloud based" processor).

Some example embodiments further comprise means for providing the mood state information to said processor, wherein the generated content is based, at least in part, on said mood state information.

The means for obtaining information relating to the mood state of the user may comprise at least one of: one or more biometric sensors; or a user interface enabling the user to provide a current or desired user mood state.

Some example embodiments further comprise means for generating data relating to distances of one or more objects in the captured imaging data. Furthermore, some example embodiments comprise means for providing the distance data to said processor, wherein the generated content is based, at least in part, on said distance data. For example, a volume of the generated content maybe dependent on said distance information.

The said means for capturing imaging data may comprise a camera (or some other imaging device). Alternatively, or in addition, the said means for capturing imaging device may comprise a LiDAR device.

The processor may be provided by a device in wireless communication with said apparatus. For example, a Bluetooth^{®} or similar connection maybe provided.

Some example embodiments further comprise means for sharing the generated content with one or more other users (e.g. co-workers of a first user).

In a second aspect, this specification describes an apparatus (e.g. a processor) comprising: means for receiving imaging data relating to a user environment captured at a user device (e.g. captured using an eyewear device); means for providing the imaging data to a data processing algorithm to generate content based, at least in part, on the captured imaging data, wherein the generated content comprises audio content; and means for providing said generated content for output. The apparatus of the second aspect maybe in communication with the apparatus of the first aspect described above (e.g. using a wireless connection, such as Bluetooth^{®}). In some example embodiments, the audio content is intended to induce an autonomous sensory meridian response (ASMR).

Some example embodiments further comprise means for receiving information relating to a mood state of the user; and means for providing the received mood state information to the data processing algorithm, wherein the generated content is based, at least in part, on said mood state information. For example, the generated content maybe personalised in some way to the respective user.

Some example embodiments further comprise means for receiving data relating to distances of one or more objects in the captured imaging data. The said generated content may be based, at least in part, on said distance data. For example, a volume of the generated content may be dependent on said distance information.

Some example embodiments further comprise means for sharing the generated content with one or more other users (e.g. co-workers of a first user).

In a third aspect, this specification describes a method comprising: capturing imaging data relating to a user environment; obtaining user information; providing at least the captured imaging data to a processor for generating content based, at least in part, on the captured imaging data, wherein the generated content comprises audio content; receiving said generated content; and providing an audio output to the user based on said generated content, wherein the audio output is dependent, at least in part, on the captured imaging data and the user information. The audio content may be modified (e.g. personalised) in some way. The audio content may be intended to induce an autonomous sensory meridian response (ASMR).

Some example embodiments further comprise obtaining information relating to a mood state of the user, wherein the output provided to the user is based, at least in part, on said mood state information. Thus, the output (e.g. audio output) provided to the user maybe personalised based (at least in part) on said mood state information.

Some example embodiments further comprise providing the mood state information to said processor, wherein the generated content is based, at least in part, on said mood state information.

Some example embodiments further comprise generating data relating to distances of one or more objects in the captured imaging data. Furthermore, some example embodiments further comprise providing the distance data to said processor, wherein the generated content is based, at least in part, on said distance data. For example, a volume of the generated content maybe dependent on said distance information. Some example embodiments further comprise sharing the generated content with one or more other users (e.g. co-workers of a first user).

In a fourth aspect, this specification describes a method comprising: receiving imaging data relating to a user environment captured at a user device; providing the imaging data to a data processing algorithm to generate content based, at least in part, on the captured imaging data, wherein the generated content comprises audio content; and providing said generated content for output. The audio content may be intended to induce an autonomous sensory meridian response (ASMR).

Some example embodiments further comprise for receiving information relating to a mood state of the user; and providing the received mood state information to the data processing algorithm, wherein the generated content is based, at least in part, on said mood state information. For example, the generated content may be personalised in some way to the respective user.

Some example embodiments further comprise receiving data relating to distances of one or more objects in the captured imaging data. The said generated content maybe based, at least in part, on said distance data. For example, a volume of the generated content maybe dependent on said distance information.

Some example embodiments further comprise means for sharing the generated content with one or more other users (e.g. co-workers of a first user).

In a fifth aspect, this specification describes computer-readable instructions which, when executed by a computing apparatus, cause the computing apparatus to perform (at least) any method as described herein (including the methods of the third and fourth aspects described above).

In a sixth aspect, this specification describes a computer-readable medium (such as a non-transitory computer-readable medium) comprising program instructions stored thereon for performing (at least) any method as described herein (including the methods of the third and fourth aspects described above).

In a seventh aspect, this specification describes an apparatus comprising: at least one processor; and at least one memory including computer program code which, when executed by the at least one processor, causes the apparatus to perform (at least) any method as described herein (including the methods of the third and fourth aspects described above).

In an eighth aspect, this specification describes a computer program comprising instructions which, when executed by an apparatus, cause the apparatus to: capture imaging data relating to a user environment; obtain user information; provide at least the captured imaging data to a processor for generating content based, at least in part, on the captured imaging data, wherein the generated content comprises audio content; receive said generated content; and provide an audio output to the user based on said generated content, wherein the audio output is dependent, at least in part, on the captured imaging data and the user information. The audio content may be intended to induce an autonomous sensory meridian response (ASMR).

In a ninth aspect, this specification describes a computer program comprising instructions which, when executed by an apparatus, cause the apparatus to: receive imaging data relating to a user environment captured at a user device; provide the imaging data to a data processing algorithm to generate content based, at least in part, on the captured imaging data, wherein the generated content comprises audio content; and provide said generated content for output. The audio content may be intended to induce an autonomous sensory meridian response (ASMR).

In a tenth aspect, this specification describes: an imaging device (or some other means) for capturing imaging data relating to a user environment; a user interface and/or biometric sensor(s) (or some other means) for obtaining user information; a controller (or some other means) for providing at least the captured imaging data to a processor for generating content based, at least in part, on the captured imaging data, wherein the generated content comprises audio content; the controller (or some other means) for receiving said generated content; and a loudspeaker (or some other means) for providing an audio output to the user based on said generated content, wherein the audio output is dependent, at least in part, on the captured imaging data and the user information.

In an eleventh aspect, this specification describes: an input (or some other means) for receiving imaging data relating to a user environment captured at a user device; a processor (or some other means) for providing the imaging data to a data processing algorithm to generate content based, at least in part, on the captured imaging data, wherein the generated content comprises audio content; and an output (or some other means) for providing said generated content for output.

### Brief description of the drawings

Example embodiments will now be described, by way of example only, with reference to the following schematic drawings, in which:
FIG. 1 is a block diagram of a system in accordance with an example embodiment;
FIG. 2 is a block diagram of a system in accordance with an example embodiment;
FIG. 3 shows an eyewear device in accordance with an example embodiment;
FIGS. 4 to 6 are flow charts showing algorithms in accordance with example embodiments;
FIG. 7 is a block diagram of a system in accordance with an example embodiment;
FIG. 8 is a block diagram of a system in accordance with an example embodiment;
FIG. 9 is a flow chart showing an algorithm in accordance with an example embodiment;
FIG. 10 is a flow chart showing an algorithm in accordance with an example embodiment;
FIG. 11 is a block diagram of a system in accordance with an example embodiment;
FIG. 12 is a flow chart showing an algorithm in accordance with an example embodiment;
FIG. 13 shows a first neural network used in some example embodiments
FIG. 14 is a block diagram of components of a system in accordance with an example embodiment; and
FIG. 15 shows an example of tangible media for storing computer-readable code which when run by a computer may perform methods according to example embodiments described above.

### Detailed description

The scope of protection sought for various embodiments of the disclosure is set out by the independent claims. The embodiments and features, if any, described in the specification that do not fall under the scope of the independent claims are to be interpreted as examples useful for understanding various embodiments of the disclosure.

In the description and drawings, like reference numerals refer to like elements throughout.

Autonomous sensory meridian response (ASMR) is a complex emotional state that some people experience when exposed to particular types of auditory or visual stimuli such as whispering, tapping, slow movements or crisp sounds. A common outcome of ASMR is the generation of a tingling sensation in the user. ASMR is also known to generate calming or relaxing sensations, or a general feeling of comfort.

A significant amount of content intended to induce an ASMR is available, for example on the Internet. Such content generally needs to be navigated manually, not least because different users experience different responses to such content. Requiring manual navigation of such content can result in a loss of the effect (e.g. a relaxing effect) that can be obtained from such content. Indeed, the abundance of the content that is available can be overwhelming.

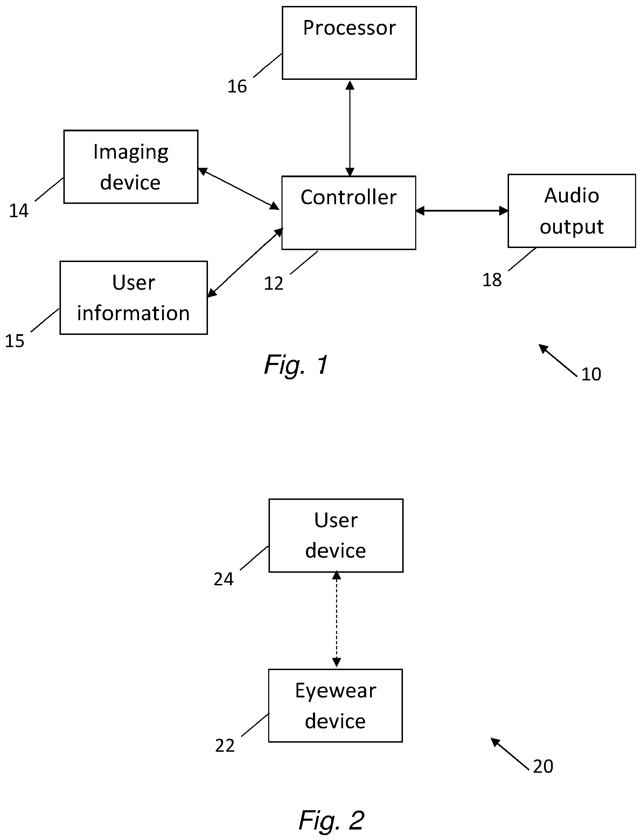

FIG. 1 is a block diagram of a system, indicated generally by the reference numeral 10, in accordance with an example embodiment. The system 10 comprises a controller 12, an imaging device 14, a user information input 15, a processor 16 and an audio output 18.

The imaging device 14 is used to capture imaging data relating to a user environment. As discussed further below, the imaging device 14 may be a camera, a Light Detection and Ranging (LiDAR) device, or some similar device.

The controller 12 receives captured imaging data from the imaging device 14 and also receives the user information input 15. The user information may, for example, relate to a mood of the user and/or one or more user preferences. As discussed further below, the user information input may be obtained from a user interface (enabling a user to provide information such as mood information or user preferences) and/or from one or more sensors (such as biometric sensors).

The controller 12 provides at least the captured imaging data to the processor 16. The processor generates audio content based, at least in part, on the captured imaging data. In some example embodiments, the processor 16 implements a machine-learning (ML) algorithm, as discussed in detail below.

The controller 12 receives the generated audio content from the processor 16 and provides audio content based on the generated audio content to the audio output 18 (e.g. using a loudspeaker). The audio content provided to the audio output 18 may be the audio content as generated by the processor 16; alternatively, the audio content as generated by the processor 16 may be modified in some way prior to being output. For example, the audio content may be modified based, at least in part, on the user information input 15. Thus, for example, the audio content may be personalised in some way to the user (e.g. to actual or desired user mood and/or to user preferences, such as a preference for metallic sounds over soft sounds). Alternatively, or in addition, the user information input 15 may be provided to the processor 16 and used in the generation of the audio content (e.g. the audio content may be personalised to the user at the processor 16).

The audio content generated by the processor 16 may be content that is intended to induce an ASMR response.

FIG. 2 is a block diagram of a system, indicated generally by the reference numeral 20, in accordance with an example embodiment. The system 20 comprises an eyewear device 22 (such as smart glasses, virtual reality glasses or the like) and a user device 24 (such as a mobile phone, computer or similar device). The eyewear device 22 and the user device 24 may be in wireless communication (as indicated by the dotted arrows). For example, the wireless communication may be a Bluetooth^{®} connection, although many other wireless communication implementations are possible.

The eyewear device 22 may be used to implement some or all of the controller 12, imaging device 14, user information input 15 and audio output 18 of the system 10 described above. The user device 24 may be used to implement the processor 16 of the system 10 described above (and may also be used to implement other aspects of the system 10, such as some or all of the user information input 15). The implementation of the processor 16 at the user device 24 is not essential to all example embodiments. For example, the processor 16 could be provided at the eyewear device or elsewhere (e.g. at a remote server).

Thus, the eyewear device 22 may be used as both an input and output peripheral device, whilst the user device 24 may be used as a principal processing unit.

The systems 10 and 20 can be used to provide ambient-informed ASMR content using an eyewear device (such as smart glasses). For example, a current surrounding environment can be recreated in ASMR sounds to promote relaxation or enhance creativity etc. The imaging device 14 (e.g. a camera or LiDAR device) on the eyewear device can be used to scan user surroundings. The images (or point clouds from the LiDAR) of the surroundings (together, for example, with an emotional state of the user) can be converted by the processor 16 into the ASMR sound they make, for instance an image of a keyboard may be converted into a sound of key-tapping, an image of a stapler into the sound of clipping, an image of leaves on trees moving outside the window into the sound of wind sound and so on. The audio signal can be sent to the eyewear device 22 for the user to enjoy. Moreover, as discussed further below, in a connected remote office scenario, employees can experience each other's surrounding by sharing generated audio content (for example to create a team-friendly environment even for remote teams, e.g. the remote team may have access to a virtual space or virtual world which may be referred to as "the metaverse").

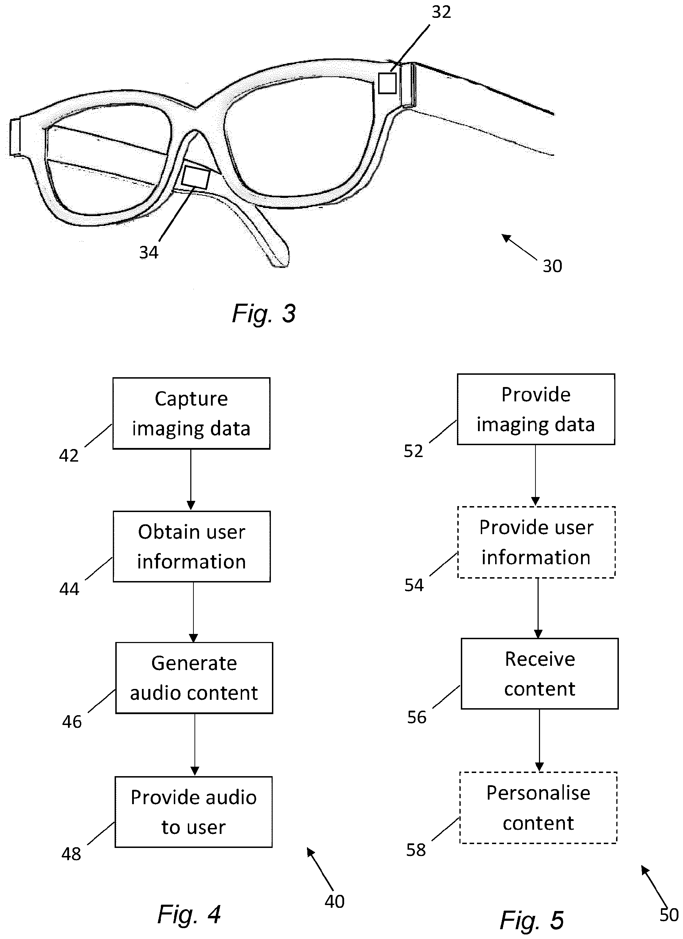

FIG. 3 shows an eyewear device, indicated generally by the reference numeral 30, in accordance with an example embodiment. The eyewear device 30 may be used to implement the eyewear device 22 described above. The eyewear device 30 may, for example, be glasses, smart-glasses, virtual reality glasses, mixed reality or augmented reality glasses, a headset, head-mounted display, or the like.

The eyewear device 30 includes an imaging device 32 (that can be used to implement the imaging device 14 of the system 10 described above) and a loudspeaker 34 (that can be used to implement the audio output 18 of the system 10 described above). A user interface (not shown) may be provided to enable user information input 15 to be provided.

FIG. 4 is a flow chart showing an algorithm, indicated generally by the reference numeral 40, in accordance with an example embodiment. The algorithm 40 may be implemented using the system 10 described above.

The algorithm 40 starts at operation 42, where imaging data relating to a user environment is captured, for example using the imaging device 14 (e.g. using the imaging device 32 of the eyewear device 30).

At operation 44, user information is obtained (e.g. the user information input 15). Note that the operations 42 and 44 may be implemented in a different order or in parallel.

At operation 46, audio content is generated based, at least in part, on the imaging data captured in the operation 42. The operation 46 maybe implemented by the processor 16.

At operation 48, an audio output (e.g. the audio output 18) is provided to the user (e.g. using the loudspeaker 34 of the eyewear device 30). The audio output is based on the audio content generated in the operation 46.

Audio provided to the user in the operation 48 may be personalised to that user. The personalisation may take place in the operation 46, the operation 48, or both. The personalisation may be based (at least in part) on a mood state of the user. For example, user mood information (e.g. a current mood or a desired user mood) may be used to inform the volume of the audio output and/or the intensity or type of the audio output. Alternatively, or in addition, the placement of an audio output within space maybe dependent on user information (such as user preferences). For example, audio (e.g. a spatial audio object) may be placed closer to the ear of the user than natural locations of captured images; such audio placement is a factor known to affect ASMR responses in some users.

FIG. 5 is a flow chart showing an algorithm, indicated generally by the reference numeral 50, in accordance with an example embodiment. The algorithm 50 is an example implementation of the operation 46 of the algorithm 40 and may be implemented by the controller 12 of the system 10. The controller 12 may form part of the eyewear device 30 described above, although this is not essential to all example embodiments - for example, the controller may be provided (at least in part) at the user device 24.

The algorithm 50 starts at operation 52, where imaging data relating to the user environment (e.g. as obtained in the operation 42) is provided, for example to the processor 16.

At operation 54, user information (e.g. as obtained in the operation 44) maybe provided (for example to the processor 16). The user information may relate to a mood state of the user. The operations 52 and 54 maybe carried out in a different order or in parallel. Alternatively, the operation 54 maybe omitted (e.g. such that the processor 16 does not make use of user information when generating audio content).

At operation 56, audio content is received. For example, the controller 12 may receive audio content as generated by the processor 16 based, at least in part, on the imaging device provided in the operation 52 (and, in some example embodiments, based on the user information provided in the optional operation 54).

In some example embodiments, the audio content received in the operation 56 is personalised to the user in optional operation 58. The personalisation may be based, at least in part, on the user information obtained in the operation 44. Thus, the audio content provided to the user in the operation 48 of the algorithm 40 may be personalised at the processor 16 based on user information provided in the operation 54, personalised in the operation 58 (e.g. at the controller 12), or both.

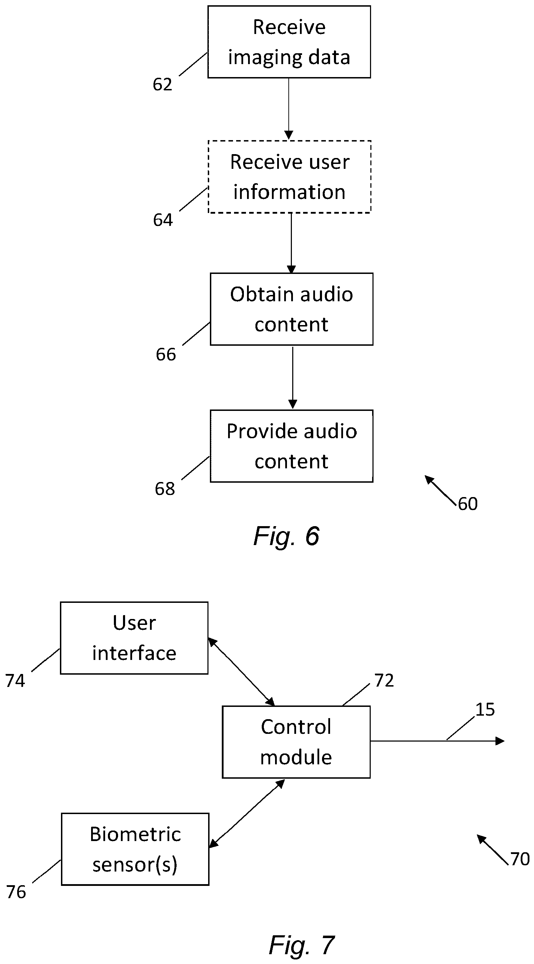

FIG. 6 is a flow chart showing an algorithm, indicated generally by the reference numeral 60, in accordance with an example embodiment. The algorithm 60 is an example implementation of the operation 46 of the algorithm 40 and may be implemented by the processor 16 of the system 10.

The algorithm 60 starts at operation 62, where imaging data relating to a user environment is received. The imaging data may be captured by the imaging device 14 and provided to the processor 16 by the controller 12 (e.g. in the operation 52 described above).

At optional operation 64, user information relating to the user may be received (for example from the controller 12, e.g. in the operation 54). The user information may relate to a mood state of the user. The operations 62 and 64 maybe carried out in a different order or in parallel. Alternatively, the operation 64 may be omitted (e.g. if the processor 16 does not make use of user information when generating audio content).

At operation 66, audio content is obtained based, at least in part, on the imaging data received in the operation 62. The audio content may be based, at least in part, on the user information (such as mood state information) obtained in the operation 64 (although the use of user information may be omitted in some example embodiments). The audio content maybe obtained in the operation 66 from a machine learning algorithm, as discussed further below.

At operation 68, the content as obtained in the operation 66 is provided (e.g. to the controller 12). The said audio may then be provided to the user in the operation 48 of the algorithm 40 described above.

FIG. 7 is a block diagram of a system, indicated generally by the reference numeral 70, in accordance with an example embodiment. The system comprises a control module 72, a user interface 74 and one or more biometric sensors 76. The control module 72 is used to provide the user information input 15 described above.

The user interface 74 enables a user to provide information for use in generating the user information input 15. For example, the user interface may enable user mood information or user preferences to be provided. The user preferences may relate to characteristics known to induce an ASMR response for the user. The user may, for example, use the user interface 74 to indicate their current mood, or a mood that they would like to be in.

The one or more biometric sensors 76 may include sensors that can be used to determine a mood state of the user. Such sensors may include heart rate monitor(s), breathing rate sensor(s), temperature sensor(s), electroencephalogram (EEG) sensor(s), photoplethysmography PPG sensor(s), galvanic skin response GSR sensor(s) inertial measurement unit (IMU) sensor(s), camera(s), microphone(s) or the like.

It should be noted that one or both of the user interface 74 and the biometric sensor(s) 76 maybe omitted in some example embodiments. The skilled person will be aware of alternative or additional means for generating the user information input 15.

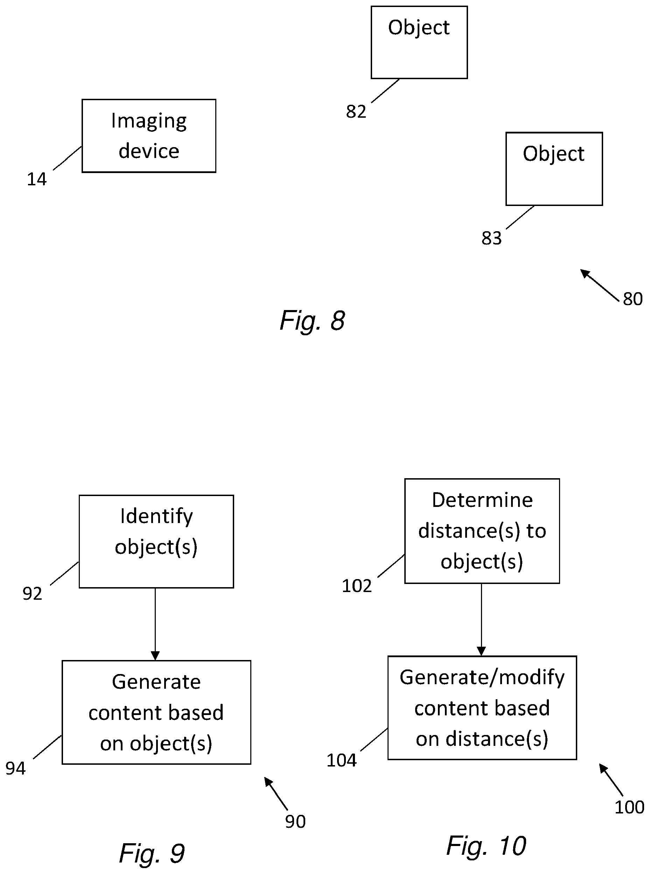

FIG. 8 is a block diagram of a scene, indicated generally by the reference numeral 80, in accordance with an example embodiment. The scene 80 comprises a first object 82 and a second object 83. The imaging device 14 described above (e.g. a camera or LiDAR device) is used to capture images of the objects 82 and 83.

The imaging device 14 may comprise means (e.g. associated software or firmware) for creating bounding boxes over one or more objects (such as images of the objects 82 and 83) in the captured imaging data. Alternatively, or in addition, the imaging device 14 may comprise means for creating a 3D point cloud (e.g. based on LiDAR data) of one or more objects in the captured imaging data. The bounding boxes and/or 3D point cloud may be used for processing the captured images (e.g. by the controller 12 and/or by the processor 16), for example in a pre-processing step that may be performed by a machine learning (ML) model, or mathematical function (such functions may, for example, be sufficiently simple that they can be implemented at an eyewear device or the like). The skilled person will be aware of alternative technologies that may be used in example imaging implementations.

FIG. 9 is a flow chart showing an algorithm, indicated generally by the reference numeral 90, in accordance with an example embodiment.

The algorithm 90 starts at operation 92, where one of more objects in a captured image (e.g. image(s) of the objects 82 and 83) are identified. At operation 94, audio content is generated based on the identified objects. By way of example, audio content may be generated to mimic the sounds of objected identified in the operation 92 in some way. For example, if a computer keyboard is identified, then a tapping noise may be generated.

FIG. 10 is a flow chart showing an algorithm, indicated generally by the reference numeral 100, in accordance with an example embodiment.

The algorithm 100 starts at operation 102, where distance(s) to one or more objects in a captured image (e.g. image(s) of the objects 82 and 83) are determined. For example, the distances of the object(s) from the user, or from some other reference point, maybe determined. At operation 104, audio content is generated or modified based on the distances. For example, a volume of generated audio content, or an effective location of the audio content, maybe dependent on the location of the respective object within a scene.

Of course, the algorithms 90 and 100 may both be used in example embodiments. For example, audio content may be generated dependent on the identity of one or more object(s) and modified based on location(s) of the object(s) within a scene.

The algorithm 90 maybe used to recognise one or more objects in a scene and within the field of view of the imaging device 14 (e.g. a stapler on a desk to the user's left side, a tree outside a window to the user's right, etc.). Based on user mood information and/or user preference(s) (which may be learned), the algorithm 90 may select and generate audio content (e.g. ASMR audio) representing the stapler for output.

In the algorithm 100, a spatial audio object maybe generated and located in the scene at the stapler's position (e.g. to provide a realistic audio effect). However, based on the user mood information and/or user preference(s), the realistic (or natural) placement may be overridden so that a location that better suits the ASMR effect is selected instead. For example, the generated audio may be played more softly or more gently and/or at a location that is closer to the user's ear. These effects and the placement for the generated spatial audio object maybe dynamically modified or updated automatically, for example as the user's mood and/or preference(s) change. It should be appreciated this is only one example- the effect(s) and the placement for a generated spatial audio object may vary at least based on to the object recognised, and the user information input 15 (e.g. the user mood information and/or preference(s)).

Moreover, the user mood information and/or preference(s) may be a factor in deciding which object is selected (e.g. stapler or tree in the example described above). For example, one object may be classified as more calming (or more energising, etc.) than another. This may be a user-specific preference and a controller (e.g. a ML model) may learn what works well for a particular user and what does not work well for that user (e.g. based on explicit feedback from the user, or if one or more sensors determine an associated physiological response is achieved or not: e.g. for a calming effect, the user's heart rate may decrease, or a GSR sensor may indicate a lower stress level of the user, etc.).

Thus, apparatus and systems described herein may be used to generate or deliver the most likely appropriate audio content to a user rather than the user needing to find that content.

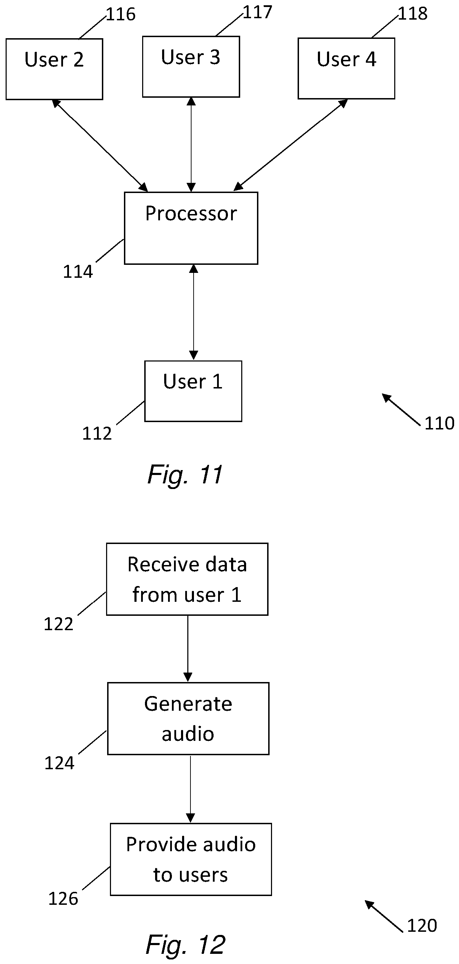

FIG. 11 is a block diagram of a system, indicated generally by the reference numeral 110, in accordance with an example embodiment. The system 110 comprises a first user 112, a processor 114, a second user 116, a third user 117 and a fourth user 118. The second to fourth users 116 to 118 may be co-workers of the first user 112. At least some of the users may be geographically remote from the others users (e.g. working from home).

The processor 114 (e.g. the processor 16 and/or a remote processor) maybe used to provide an audio output to the first user 112 based on imaging data captured at/by the first user and user information relating to the first user. The audio output provided to the first user 112 may be generated in accordance with the principles described above.

The processor 114 may share the audio content generated for the first user 112 with some or all of the second to fourth users 116 to 118. Thus, for example, audio content relating to the surroundings of the first user may be shared with the other users (e.g. co-workers of the first user).

FIG. 12 is a flow chart showing an algorithm, indicated generally by the reference numeral 120, in accordance with an example embodiment. The algorithm 120 may be implemented using the system 110 described above.

The algorithm 120 starts at operation 122, where data relating to the first user 112 (e.g. as captured by a device, such as an eyewear device, at the first user) is obtained. The data obtained may include imaging data and may also include user information data relating to the first user (as discussed in detail above).

At operation 124, audio is generated based, at least in part, on the data received in the operation 122.

At operation 126, the audio is provided to first user 112 and also to one or more of the second to fourth users 116 to 118. Note that the audio provided to one or more of the second to fourth users may, in some example embodiments, be personalised to the respective user(s) in some way.

For example, the first user 112 may be located in an office and one or more of the other users may be working remotely (e.g. at home). Office based sounds may be generated by the processor 114 (based on imaging data captured of the first user's office environment) and shared with the other users (e.g. co-workers).

Alternatively, a co-worker living in the city might want the calming sounds of a country house or kitchen sounds of another co-worker, or the other way around. Such collaboration may assist with the creation of a more inclusive remote office where co-workers can feel as if they are co-located using ASMR effects.

In example implementations, the processor 16 may incorporate a machine learning (ML) algorithm (or ML model). The ML algorithm may be implemented at a user device (such as the user device 24) or in some other location (such as at a server). The ML algorithm may be used to receive inputs such as one or more of: images captured by a camera or some other imaging device; point clouds and/or distance data obtained by a LiDAR device; current or desired user mood information; biometric sensor data; and other user information. The ML model generates audio content based on the inputs (e.g. multi-modal inputs). Distance to objects in an image may be used as a modality to inform the volume of the audio sound relating to a captured object. User information, such as the user mood information and/or user preference(s) may be used to inform an intensity and/or type of audio generated. Alternatively, or in addition, the mood information, user preference(s) and/or some other user information maybe used to change a colour of visual content provided to a user, e.g. via a display of the eyewear device 22. The colour change maybe applied to individually recognised object(s) in the scene. For instance, a red colour effect (e.g. a transparent overlay) may be displayed based on the user mood information indicating the user is "angry". As the user's mood changes (for example, if the user starts to feel calmer), the settings may be configured so that the displayed colour changes to a different shade or different colour, for example depending on a colour palette that is pre-selected by the user. The colour alteration can be part of the ML model or a different module which separately uses the user information, such as the user mood information and/or user preference(s), to alter the colour after the ML model inference stage. The mood change(s) (or user preference change(s)) may occur at a different frequency to the image and/or LiDAR inputs, so a separate module for the user mood data and/or the user preference(s) data may bring computational benefits.

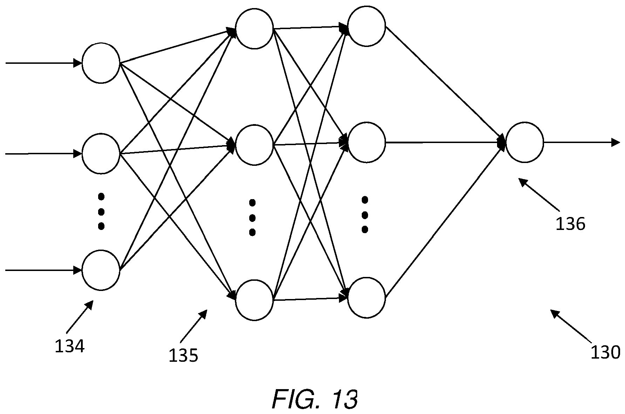

FIG. 13 shows a neural network, indicated generally by the reference numeral 130, used in some example embodiments. The neural network 130 may be used to implement the ML algorithm described above.

The neural network 130 comprises an input layer 134, one or more hidden layers 135, and an output layer 136. At the input layer 134, data such as imaging device outputs and user information are received as input(s). The hidden layers 135 may comprise a plurality of hidden nodes. At the output layer 136, audio data may be provided as an output. The neural network 130 may be any type of deep neural network. This can be a multi-input multi-output model where the data is organised in such a way that they are all considered as one, and the labels (as described below) are merged into multidimensional labels.

The neural network 130 may be a multi-stage neural network where, in the first stage the object is identified, in the second stage the user mood is identified, and third stage the association of the inputs (which are outputs from previous stages) to the ASMR audio (and/or the colour change of visual content) for output to the user. The different stages may be completely separate deep learning models, or may be multi-head models where each head has distinctive features for the specific classification and merging features deeper in the model.

Depending on the type of neural network 130 chosen for a particular application, the cost function maybe: a traditional single cost function such as cross-entropy, or a weighted-sum cost function depending on the weight of each model, if using different models.

The neural network may be trained based on machine-learning principles. For example, and as detailed below, training data may be generated that is known to induce an ASMR effect on at least some users and used to match audio outputs to various scenes.

The ML model may be trained based on publicly available or proprietary data, at least some of which may be related to a particular user. In some examples, as input for the training procedure, at least one of images, image data or LiDAR data can be used. Datasets with these types of input may be readily found on the Internet (publicly available). Proprietary (or user-specific) data may be obtained from at least one of a remote server, one or more other devices, or from the eyewear device 22 or user device 24. Other inputs for the training procedure may optionally include the user information, such as the user mood information and/or the user preference(s). Alternatively, or in addition, the ML model may be updated in use, based on actual responses of the user. For example, a generic ML model may be generated based on training data for many users, with that model being updated in use for an individual user. This training procedure may be performed for the generic ML model on the cloud (e.g. a remote server, or (an)other connected one or more devices, which may or may not include eyewear device 22 or user device 24). Alternatively, or in addition, to protect the privacy of a user's data, a training procedure that is personalized to the user maybe performed at the eyewear device 22, the user device 24, or managed between both of these devices. Nevertheless, if, for example, the cloud is a trusted entity, the user-specific data may, in some example embodiments, be sent to the cloud for personalised training. In examples of the disclosure where the generic ML model is applied, the user information (such as the user mood information and/or user preference(s)) may be used as a filter after the ML model inference. For example, the ML model may infer various types of sounds such as sounds classified as metallic, sharp, or smooth. However, if the user has specified a preference for metallic sounds, for example, the other types of sounds may be filtered out and not generated or not output at the eyewear device 22 or user device 24.

The audio content generated in example embodiments described above may be derived from, or modified from, a wide range of audio data (e.g. ASMR audio content) that is currently available. For example, many ASMR and other audio content examples could be scraped, for example using an application programming interface (API), from Internet resources (such as YouTube^{®}). There is a significant amount of such existing audio content that can be obtained for use as labels, as part of the training phase, and optionally re-used or modified for the subsequent generated audio output e.g. as an output of the ML model at inference, based on the learning from the training phase and given inputs to the ML model, as described above. Alternatively, or in addition, labels for the training phase may be obtained through an audio synthesis process, for example using one or more online general adversarial networks (GANs); see for example https://magenta.tensorflow.org/gansynth. The synthesised audio content may later be used as the generated audio content, e.g. as an output of the ML model at inference, based on the learning from the training phase and given inputs to the ML model, as described above.

Although the invention has generally been described in relation to ASMR, the principles described herein are not limited to such applications. In particular, the principles described herein can be used in the selection, generation or provision of audio content based on other factors and requirements.

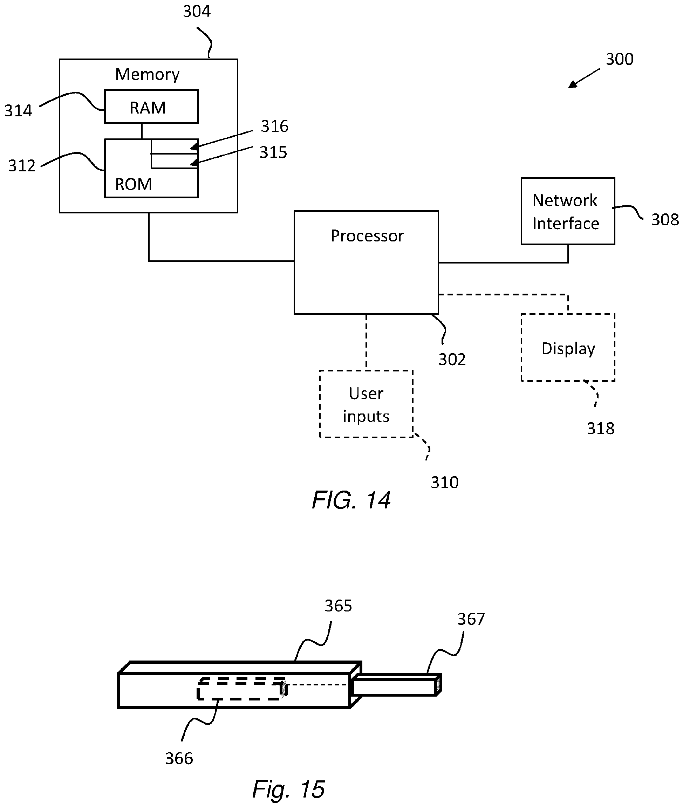

For completeness, FIG. 14 is a schematic diagram of components of one or more of the example embodiments described previously, which hereafter are referred to generically as a processing system 300. The processing system 300 may, for example, be (or may include) the apparatus referred to in the claims below.

The processing system 300 may have a processor 302, a memory 304 coupled to the processor and comprised of a random access memory (RAM) 314 and a read only memory (ROM) 312, and, optionally, a user input 310 and a display 318. The processing system 300 may comprise one or more network/apparatus interfaces 308 for connection to a network/apparatus, e.g. a modem which may be wired or wireless. The network/apparatus interface 308 may also operate as a connection to other apparatus such as device/apparatus which is not network side apparatus. Thus, direct connection between devices/apparatus without network participation is possible.

The processor 302 is connected to each of the other components in order to control operation thereof.

The memory 304 may comprise a non-volatile memory, such as a hard disk drive (HDD) or a solid state drive (SSD). The ROM 312 of the memory 304 stores, amongst other things, an operating system 315 and may store software applications 316. The RAM 314 of the memory 304 is used by the processor 302 for the temporary storage of data. The operating system 315 may contain code which, when executed by the processor implements aspects of the methods and algorithms 40, 50, 60, 90, 100 and 120 described above. Note that in the case of small device/apparatus the memory can be most suitable for small size usage i.e. not always a hard disk drive (HDD) or a solid state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts maybe all inside device/apparatus such as IoT device/apparatus i.e. embedded to very small size.

In some example embodiments, the processing system 300 may also be associated with external software applications. These maybe applications stored on a remote server device/apparatus and may run partly or exclusively on the remote server device/apparatus. These applications maybe termed cloud-hosted applications. The processing system 300 maybe in communication with the remote server device/apparatus in order to utilize the software application stored there.

FIG. 15 shows tangible media, specifically a removable memory unit 365, storing computer-readable code which when run by a computer may perform methods according to example embodiments described above. The removable memory unit 365 may be a memory stick, e.g. a USB memory stick, having internal memory 366 for storing the computer-readable code. The internal memory 366 may be accessed by a computer system via a connector 367. Other forms of tangible storage media may be used. Tangible media can be any device/apparatus capable of storing data/information which data/information can be exchanged between devices/apparatus/network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" maybe any non-transitory media or means that can contain, store, communicate, propagate or transport the instructions for use by or in connection with an instruction execution system, apparatus, or device, such as a computer.

Reference to, where relevant, "computer-readable medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices/apparatus and other devices/apparatus. References to computer program, instructions, code etc. should be understood to express software for a programmable processor firmware such as the programmable content of a hardware device/apparatus as instructions for a processor or configured or configuration settings for a fixed function device/apparatus, gate array, programmable logic device/apparatus, etc.

If desired, the different functions discussed herein maybe performed in a different order and/or concurrently with each other. Furthermore, if desired, one or more of the above-described functions may be optional or may be combined. Similarly, it will also be appreciated that the flow diagrams of FIGS. 4 to 6, 9, 10 and 12 are examples only and that various operations depicted therein may be omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

Although various aspects of the invention are set out in the independent claims, other aspects of the invention comprise other combinations of features from the described example embodiments and/or the dependent claims with the features of the independent claims, and not solely the combinations explicitly set out in the claims.

It is also noted herein that while the above describes various examples, these descriptions should not be viewed in a limiting sense. Rather, there are several variations and modifications which may be made without departing from the scope of the present invention as defined in the appended claims.

## Claims

1. An apparatus comprising:
means for capturing imaging data relating to a user environment;
means for obtaining user information;
means for providing at least the captured imaging data to a processor for generating content based, at least in part, on the captured imaging data, wherein the generated content comprises audio content;
means for receiving said generated content; and
means for providing an audio output to the user based on said generated content, wherein the audio output is dependent, at least in part, on the captured imaging data and the user information.

2. An apparatus as claimed in claim 1, further comprising:
means for obtaining information relating to a mood state of the user, wherein the output provided to the user is based, at least in part, on said mood state information.

3. An apparatus as claimed in claim 2, further comprising:
means for providing the mood state information to said processor, wherein the generated content is based, at least in part, on said mood state information.

4. An apparatus as claimed in claim 2 or claim 3, wherein the means for obtaining information relating to the mood state of the user comprises at least one of:
one or more biometric sensors; or
a user interface enabling the user to provide a current or desired user mood state.

5. An apparatus as claimed in any one of the preceding claims, further comprising:
means for generating data relating to distances of one or more objects in the captured imaging data.

6. An apparatus as claimed in claim 5, further comprising:
means for providing the distance data to said processor, wherein the generated content is based, at least in part, on said distance data.

7. An apparatus as claimed in any one of the preceding claims, wherein the means for capturing imaging data comprises at least one of:
a camera; or
a LiDAR device.

8. An apparatus as claimed in any one of the preceding claims, wherein said processor is provided by a device in wireless communication with said apparatus.

9. An apparatus as claimed in any one of the preceding claims, wherein the apparatus is an eyewear device.

10. An apparatus comprising:
means for receiving imaging data relating to a user environment captured at a user device;
means for providing the imaging data to a data processing algorithm to generate content based, at least in part, on the captured imaging data, wherein the generated content comprises audio content; and
means for providing said generated content for output.

11. An apparatus as claimed in claim 10, further comprising:
means for receiving information relating to a mood state of the user; and
means for providing the received mood state information to the data processing algorithm, wherein the generated content is based, at least in part, on said mood state information.

12. An apparatus as claimed in any one of the preceding claims, wherein said audio content is intended to induce an autonomous sensory meridian response.

13. An apparatus as claimed in any one of the preceding claims, further comprising:
means for sharing the generated content with one or more other users.

14. A method comprising:
capturing imaging data relating to a user environment;
obtaining user information;
providing at least the captured imaging data to a processor for generating content based, at least in part, on the captured imaging data, wherein the generated content comprises audio content;
receiving said generated content; and
providing an audio output to the user based on said generated content, wherein the audio output is dependent, at least in part, on the captured imaging data and the user information.

15. A computer program comprising instructions which, when executed by an apparatus, cause the apparatus to:
capture imaging data relating to a user environment;
obtain user information;
provide at least the captured imaging data to a processor for generating content based, at least in part, on the captured imaging data, wherein the generated content comprises audio content;
receive said generated content; and
provide an audio output to the user based on said generated content, wherein the audio output is dependent, at least in part, on the captured imaging data and the user information.
